# EUROPEAN PATENT SPECIFICATION

(11) **EP 3 924 151 B1**
(45) Date of publication and mention of the grant of the patent: **12.07.2023**
(21) Application number: 20756245.5
(22) Date of filing: 12.02.2020
(51) Int. Cl.: A61B 34/30, A61B 34/00, A61B 17/29, A61B 17/00, A61B 34/37

(54) **SURGICAL ROBOTIC SYSTEMS**
CHIRURGISCHE ROBOTISCHE SYSTEME
SYSTÈMES CHIRURGICAUX ROBOTISÉS

(30) Priority: 15.02.2019 US 201962806302 P
(43) Date of publication of application: 22.12.2021
(73) Proprietor: Covidien LP, Mansfield, MA 02048 (US)
(72) Inventor: KAPADIA, Jaimeen, Cambridge, Massachusetts 02139 (US); MISHRA, Ranjan K., Orange, Connecticut 06477 (US)
(74) Representative: Maschio, Antonio
(86) International application number: PCT/US2020/017865
(87) International publication number: WO 2020/167906

(56) References cited:
- WO-A1-2016/043845
- WO-A1-2017/205311
- WO-A1-2017/205481
- WO-A1-2017/205481
- WO-A1-2019/082224
- WO-A1-2019/191396
- WO-A1-2019/217353
- WO-A1-2020/009831
- WO-A1-2020/009831
- WO-A2-2012/068156
- US-A1- 2013 123 783
- US-A1- 2013 123 783

## Description

### BACKGROUND

Surgical robotic systems have been used in minimally invasive medical procedures. Some surgical robotic systems included a console supporting a surgical robotic arm and a surgical instrument having at least one end effector (e.g., forceps or a grasping tool) mounted to the robotic arm. The robotic arm provided mechanical power to the surgical instrument for its operation and movement.

Manually-operated surgical instruments often included a handle assembly for actuating the functions of the surgical instrument. However, when using a robotic surgical system, no handle assembly was typically present to actuate the functions of the end effector. Accordingly, to use each unique surgical instrument with a robotic surgical system, an instrument drive unit was used to interface with the selected surgical instrument to drive operations of the surgical instrument.

The instrument drive unit was typically coupled to the robotic arm via a slide. The slide allowed the instrument drive unit and the attached surgical instrument to move along an axis of the slide, providing a means for adjusting the axial position of the end effector of the surgical instrument. Patent application WO 2012/068156 describes a surgical assembly with first and second input motions, US 2013/123783 describes a surgical device with multiple motors and corresponding shafts and gears, WO 2020/009831 describes a surgical drive unit with multiple drive motors and corresponding drive shafts and drive gears, WO 2019/082224 describes a surgical systems with multiple motors and gears and WO 2019/191396 describes a surgical instrument with multiple motors and gears.

### SUMMARY

In one aspect is provided an instrument drive unit for use in a robotic surgical system, the instrument drive unit comprising:
a carriage configured to be coupled to a robotic arm;
a plurality of drive shafts rotationally supported in the carriage, the plurality of drive shafts configured for interfacing with a corresponding driven member of an electromechanical surgical instrument;
a plurality of drive gears, each drive gear of the plurality of drive gears fixed to a corresponding drive shaft of the plurality of drive shafts;
a plurality of motors; and
a plurality of motor gears each motor gear of the plurality of motor gears operably coupled to a corresponding motor of the plurality of motors, wherein each motor gear of the plurality of motor gears is configured to rotate a corresponding drive gear of the plurality of drive gears in response to an activation of a respective motor of the plurality of motors to actuate a function of the electromechanical surgical instrument, further comprising a plurality of ring gears, each ring gear of the plurality of ring gears operably coupling a corresponding motor gear of the plurality of motor gears with a corresponding drive gear of the plurality of drive gears, further comprising a plurality of drive belts, each drive belt of the plurality of drive belts disposed about a corresponding motor gear of the plurality of motor gears and a corresponding ring gear of the plurality of ring gears to operably couple the motor gears with the ring gears, characterised wherein at least a first ring gear of the plurality of ring gears has gear teeth on an inner periphery thereof and on an outer periphery thereof, wherein the gear teeth on the inner periphery of the first ring gear interface with a corresponding drive gear of the plurality of drive gears, and the gear teeth on the outer periphery of the first ring gear interface with a corresponding drive belt of the plurality of drive belts.

The plurality of ring gears may be are vertically stacked.

The unit may have a first ring gear of the plurality of ring gears and a first drive gear of the plurality of drive gears operably coupled to one another and aligned along a first plane, and wherein a second ring gear of the plurality of ring gears and a second drive gear of the plurality of drive gears operably coupled to one another and aligned along a second plane, vertically displaced from the first plane.

The plurality of ring gears may be independently rotatable relative to one another.

The plurality of drive gears may be vertically and horizontally offset from one another.

The instrument drive unit may have the plurality of motor gears vertically and horizontally offset from one another.

The instrument drive unit may further comprise a plurality of motor shafts, each motor shaft of the plurality of motor shafts extending from a corresponding motor of the plurality of motors, each motor gear of the plurality of motor gears being fixed to a corresponding motor shaft of the plurality of motor shafts.

Further details and aspects of exemplary embodiments of the present disclosure are described in more detail below with reference to the appended figures.

As used herein, the terms parallel and perpendicular are understood to include relative configurations that are substantially parallel and substantially perpendicular up to about + or - 10 degrees from true parallel and true perpendicular.

As used herein, the term "vertical" is understood to be a direction that is parallel to a longitudinal axis of the instrument drive unit. The term "horizontal" is understood to be a direction that is perpendicular to the longitudinal axis of the instrument drive unit.

### BRIEF DESCRIPTION OF THE DRAWINGS

Embodiments of the present disclosure are described herein with reference to the accompanying drawings, wherein:
FIG. 1 is a schematic illustration of a surgical robotic system including an instrument drive unit coupled to a slide in accordance with the present disclosure;
FIG. 2 is a perspective view of the instrument drive unit of the surgical robotic system of FIG. 1 with parts of a carriage of the instrument drive unit shown in phantom, illustrating internal components of the instrument drive unit;
FIG. 3 is a top, perspective view of the internal components of the instrument drive unit of FIG. 2; and
FIG. 4 is a front view of the internal components of the instrument drive unit of FIG. 3.

### DETAILED DESCRIPTION

Embodiments of the presently disclosed surgical robotic system and instrument drive units thereof are described in detail with reference to the drawings, in which like reference numerals designate identical or corresponding elements in each of the several views. As used herein, the term "distal" refers to that portion of the surgical robotic system or component thereof that is closest to the patient, while the term "proximal" refers to that portion of the surgical robotic system or component thereof further from the patient.

As will be described in detail below, provided is an instrument drive unit of a surgical robotic system configured to allow for a bottom-loading of a surgical instrument. The instrument drive unit has a plurality of drive shafts each configured to be coupled to a corresponding driven member of the surgical instrument for carrying out a discrete function of the surgical instrument. The drive shafts of the instrument drive unit are operably coupled to a discrete motor of the instrument drive unit via a plurality of gears and drive belts. The configuration of the gears and drive belts allows for a reduction in the overall height of the instrument drive unit (e.g., the instrument drive unit is more compact). Other features and benefits of the disclosed instrument drive units are further detailed below.

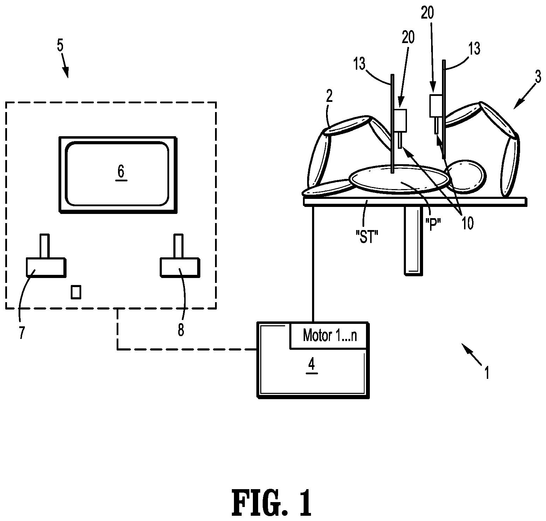

Referring initially to FIG. 1, a surgical system, such as, for example, a surgical robotic system 1, generally includes a plurality of surgical robotic arms 2, 3; an elongated slide 13 coupled to an end of each of the robotic arms 2, 3; an instrument drive unit 20 and an electromechanical instrument 10 removably attached to the slide 13 and configured to move along the slide 13; a control device 4; and an operating console 5 coupled with control device 4. The operating console 5 includes a display device 6, which is set up in particular to display three-dimensional images; and manual input devices 7, 8, by means of which a person (not shown), for example a surgeon, is able to telemanipulate robotic arms 2, 3 in a first operating mode, as known in principle to a person skilled in the art.

Each of the robotic arms 2, 3 may be composed of a plurality of members, which are connected through joints. Robotic arms 2, 3 may be driven by electric drives (not shown) that are connected to control device 4. Control device 4 (e.g., a computer) is set up to activate the drives, in particular by means of a computer program, in such a way that robotic arms 2, 3, the attached instrument drive units 20, and thus electromechanical instrument 10 execute a desired movement according to a movement defined by means of manual input devices 7, 8. Control device 4 may also be set up in such a way that it regulates the movement of the instrument drive unit 20 along the slide 13, movement of the robotic arms 2, 3, and/or movement of the drives.

Surgical robotic system 1 is configured for use on a patient "P" lying on a surgical table "ST" to be treated in a minimally invasive manner by means of a surgical instrument, e.g., electromechanical instrument 10. Surgical robotic system 1 may also include more than two robotic arms 2, 3, the additional robotic arms likewise being connected to control device 4 and being telemanipulatable by means of operating console 5. A surgical instrument, for example, an electromechanical surgical instrument 10 (including an electromechanical end effector), may also be attached to the additional robotic arm.

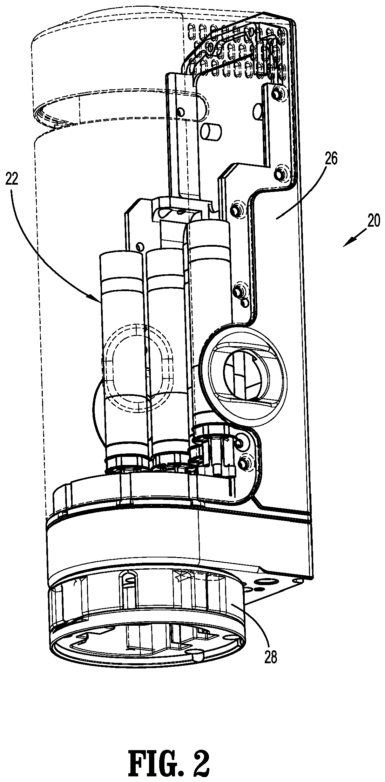

Control device 4 may control a plurality of motors, e.g., motors (Motor 1 ...n), with each motor configured to drive movement of robotic arms 2, 3 in a plurality of directions. Further, control device 4 may control a plurality of drive motors 22 (FIG. 2) of the instrument drive unit 20 to drive various operations of the surgical instrument 10. The instrument drive unit 20 transfers power and actuation forces from its motors to driven members (not shown) of the electromechanical instrument 10 to ultimately drive movement of components of the end effector of the electromechanical instrument 10, for example, a movement of a knife blade (not shown) and/or a closing and opening of jaw members of the end effector.

For a detailed description of the construction and operation of a robotic surgical system, reference may be made to U.S. Patent No. 8,828,023, entitled "Medical Workstation".

With reference to FIGS. 2-4, the instrument drive unit 20 will now be described in detail. The instrument drive unit 20 includes a carriage 26 and a coupling or sleeve 28 rotatably coupled to a distal end portion of the carriage 26 for connecting a surgical instrument 10 (FIG. 1) to the instrument drive unit 20. The carriage 26 of the instrument drive unit 20 is configured to be slidably coupled to a linear track (not shown) defined longitudinally along the slide 13 (FIG. 1). The carriage 26 houses a plurality of drive motors 22a, 22b, 22c, 22d, 22d, 22e (collectively referred herein as "22") for carrying out various functions of an attached surgical instrument. The sleeve 28 is configured to non-rotationally retain the main body portion of the surgical instrument 10 therein. Accordingly, when the surgical instrument 10 is coupled to the instrument drive unit 20, a rotation of the sleeve 28 results in a rotation of the attached surgical instrument 28.

The motors 22 of the instrument drive unit 20 are concealed within the carriage 26. The drive motors 22 are circumferentially spaced from one another and are independently actuatable via the control device 4 (FIG. 1). One of the drive motors, such as, for example, drive motor 22e, is configured to effectuate a rotation of the surgical instrument 10 when the surgical instrument 10 is coupled to the instrument drive unit 20, and the remaining drive motors 22a, 22b, 22c, 22d are configured to actuate functions of the surgical instrument 10. The drive motors 22 may be cylindrical or pancake motors. Other types of motors are also contemplated. While the instrument drive unit 20 is illustrated as having five drive motors, it is contemplated that the instrument drive unit 20 may have more or less than five drive motors.

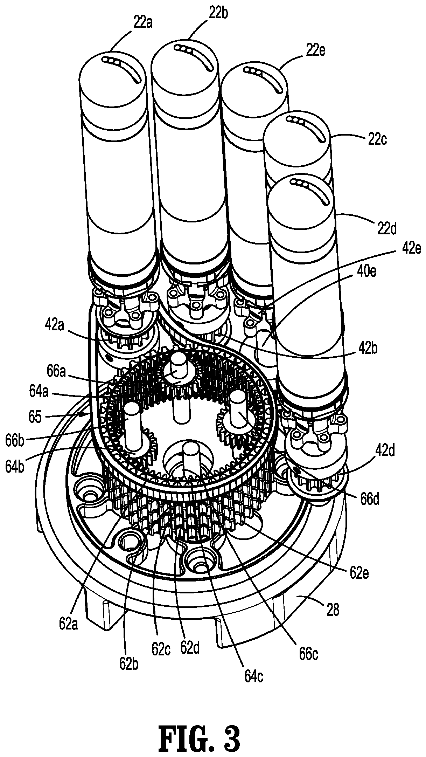

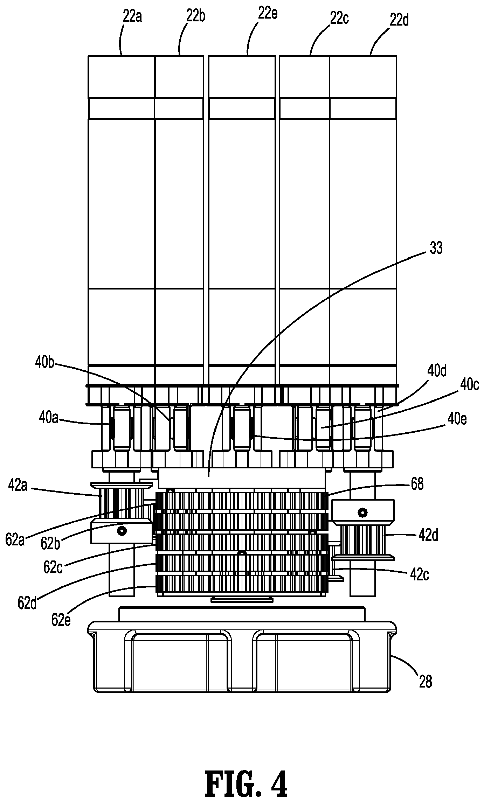

With reference to FIGS. 3 and 4, four drive motors 22a, 22b, 22c, 22d each have a rotatable motor shaft 40a, 40b, 40c, 40d, 40e (collectively referred to herein as "40") extending distally therefrom. The motor shafts 40 are circumferentially spaced from one another and each has a motor gear 42a, 42b, 42c, 42d, 42e (collectively referred to herein as "42"), such as, for example, a spur gear, rotationally fixed thereabout. Each of the motor gears 42 are positioned at a discrete vertical location on their respective motor shaft 40, such that the motor gears 42 are vertically offset a selected distance from one another. Since the motor gears 42, in addition to be vertically offset from one another, are also circumferentially spaced from one another, the motor gears 42 are offset from one another in all three dimensions.

The instrument drive unit 20 further includes a plurality of ring gears 62a, 62b, 62c, 62d, 62e (collectively referred to herein as "62"). Each of the ring gears 62 has gear teeth 68 extending from both an inner periphery thereof and an outer periphery thereof. The gear teeth 68 on the outer periphery of each of the ring gears 62 interfaces with a corresponding drive belt 65 and the gear teeth 68 on the inner periphery of each of the ring gears 62 interfaces with a corresponding drive gear of a plurality of drive gears 64a, 64b, 64c, 64d (collectively referred to herein as "64"), as will be described. In embodiments, each of the rings gears 62 may be constructed from inner and outer ring gears integrally formed with one another. While only one drive belt 65 is illustrated, it is contemplated that each ring gear 62 and motor gear 42 has a corresponding drive belt disposed thereabout. The drive belt 65 may be flexible or rigid. In embodiments, instead of having teeth, the drive belt 65 may frictionally engage the outer periphery of the ring gears 62 and the motor gears 42.

The instrument drive unit 20 further includes a plurality of drive shafts 66a, 66b, 66c, 66d (collectively referred to herein as "66"). A distal end portion of each of the drive shafts 66 is configured to operably couple to a driven member (not explicitly shown) of the surgical instrument 10. For example, the distal end portion of each of the drive shafts 66 may have a coupler (e.g., a gear) for coupling with a corresponding coupler of a driven member of the surgical instrument 10. Accordingly, upon bottom-loading of the electromechanical instrument 10 into the instrument drive unit 20, the distal end portions of the drive shafts 66 of the instrument drive unit 20 operably couple to the gears/couplers in a distal end of the main body portion (not shown) of the electromechanical instrument 10, such that a rotation of each drive shaft 66 rotates a correspondingly coupled driven member of the surgical instrument 10 to effectuate a discrete function of the surgical instrument (e.g., opening/closing of the end effector, articulation of the end effector, etc.)

The drive shafts 66 each have a drive gear 64 such as, for example, a spur gear, rotationally fixed thereabout. Each of the drive gears 64 are positioned at a discrete vertical location on their respective drive shaft 66, such that the drive gears 64 are vertically offset a selected distance from one another. Since the drive gears 64, in addition to being vertically offset, are also circumferentially spaced from one another, the drive gears 64 are offset from one another in all three dimensions. As mentioned above, the drive gears 64 each interface or intermesh with the gear teeth 68 on the inner periphery of a corresponding ring gear 62 and receive torque therefrom originating from the respective motor 22.

The instrument drive unit 20 may include a slip ring 33 (FIG. 4) received disposed adjacent the proximal-most ring gear 62a. The slip ring 33 transfers electrical signals or power between fixed structures (e.g., the drive motors 22) and rotating structures (e.g., the electromechanical surgical instrument 10). The electrical signals transferred by the slip ring 33 may be feedback signals from the electromechanical surgical instrument 10 relating to the status and location of the surgical instrument 10 and/or the status and location of adjacent tissue structures. For example, the feedback may include the temperature of the surgical instrument 10, forces experienced by the surgical instrument 10, and/or the position of certain structures of the surgical instrument 10 relative to one another or relative to the adjacent tissue structures.

Further details regarding the instrument drive unit 20 may be found in unpublished U.S. Provisional Application No. 62/693,488, filed on July 3, 2018.

In operation, the electromechanical instrument 10 is coupled to the instrument drive unit 20 by passing the main body portion of the electromechanical instrument 10 through the sleeve 28 of the instrument drive unit 20 in a proximal direction. With the main body portion of the electromechanical instrument 10 attached to the sleeve 28 of the instrument drive unit 28, the distal end portion of each of the drive shafts 66 interfaces with corresponding gears/couplers (not shown) in the proximal end of the main body portion of the electromechanical instrument 10.

To actuate a particular function of the surgical instrument 10, such as, for example, an opening or closing of an end effector of the surgical instrument 10, one of the drive motors 22 of the instrument drive unit 20, such as the first drive motor 22a, is activated via the control device 4 (FIG. 1). An activation of the first drive motor 22a rotates the first motor shaft 40a. Rotation of the first motor shaft 40a transfers torque from the first motor shaft 40a to a first driven member of the electromechanical instrument 10.

In particular, the first motor gear 42a rotates with the first motor shaft 40a, which, in turn, rotates the first drive belt 65. Rotation of the first drive belt 65 drives a rotation of the first ring gear 62a, which, in turn, rotates the first drive gear 64a. Since the first drive gear 64a is rotationally fixed about the first drive shaft 66a, and the distal end portion of the first drive shaft 66a is operably coupled to the proximal end of the first driven member of the surgical instrument 10 (FIG. 1), a rotation of the first drive gear 64a causes the first drive shaft 66a to rotate, thereby rotating the first driven member of the electromechanical instrument 10 to actuate an associated function of the surgical instrument 10. The drive motor 22e may be configured to resist rotation of the motor shaft 40e thereof during actuation of any of the drive motors 22a, 22b, 22c, 22d so that actuation of one of the drive motors 22a, 22b, 22c, 22d does not inadvertently result in a rotation of the sleeve 28.

To rotate the electromechanical instrument 10 about its longitudinal axis, the fifth drive motor 22e of the instrument drive unit 20 is activated by the control device 4 (FIG. 1). As noted above, an activation of the fifth drive motor 22e rotates the sleeve 28. Given that the electromechanical instrument 10 is non-rotationally supported in the sleeve 28, the electromechanical instrument 10 rotates with the sleeve 28 relative to the carriage 26 to change a rotational orientation of the electromechanical instrument 10. The drive motors 22a, 22b, 22c, 22d may be configured to concurrently rotate the motor shafts 40a, 40b, 40c, 40d, and in turn the drive gears 64a, 64b, 64c, 64d, with the sleeve 28 rotation. This would prevent rotation of the drive shafts 66a, 66b, 66c, 66d relative to the ring gears 62a, 62b, 62c, 62d during rotation of the sleeve 28, which may otherwise occur if the drive gears 64a, 64b, 64c, 64d were allowed to rotate relative to the ring gears 62a, 62b, 62c, 62d during rotation of the sleeve 28.

As can be appreciated, the instrument drive unit 20 described above improves usability of the surgical robotic system 1, reduces a foot-print of the overall system 1, improves safety architecture, reduces the time required to remove surgical instruments in case of an emergency, and simplifies the electronics used in the instrument drive unit 20.

It will be understood that various modifications may be made to the embodiments disclosed herein. Therefore, the above description should not be construed as limiting, but merely as exemplifications of various embodiments. Those skilled in the art will envision other modifications within the scope of the claims appended thereto.

## Claims

1. An instrument drive unit (20) for use in a robotic surgical system, the instrument drive unit comprising:
a carriage (26) configured to be coupled to a robotic arm;
a plurality of drive shafts (66a, 66b, 66c, 66d) rotationally supported in the carriage, the plurality of drive shafts configured for interfacing with a corresponding driven member of an electromechanical surgical instrument;
a plurality of drive gears (64a, 64b, 64c, 64d), each drive gear of the plurality of drive gears fixed to a corresponding drive shaft of the plurality of drive shafts;
a plurality of motors (22a, 22b, 22c, 22d); and
a plurality of motor gears (42), each motor gear of the plurality of motor gears operably coupled to a corresponding motor of the plurality of motors, wherein each motor gear of the plurality of motor gears is configured to rotate a corresponding drive gear of the plurality of drive gears in response to an activation of a respective motor of the plurality of motors to actuate a function of the electromechanical surgical instrument, further comprising a plurality of ring gears (62a, 62b, 62c, 62d) , each ring gear of the plurality of ring gears operably coupling a corresponding motor gear of the plurality of motor gears with a corresponding drive gear of the plurality of drive gears, further comprising a plurality of drive belts, each drive belt of the plurality of drive belts (65) disposed about a corresponding motor gear of the plurality of motor gears and a corresponding ring gear of the plurality of ring gears to operably couple the motor gears with the ring gears, characterised wherein at least a first ring gear of the plurality of ring gears has gear teeth on an inner periphery thereof and on an outer periphery thereof, wherein the gear teeth on the inner periphery of the first ring gear interface with a corresponding drive gear of the plurality of drive gears, and the gear teeth on the outer periphery of the first ring gear interface with a corresponding drive belt of the plurality of drive belts.

2. The instrument drive unit according to claim 1, wherein the plurality of ring gears are vertically stacked.

3. The instrument drive unit according to claim 2, wherein a first ring gear of the plurality of ring gears and a first drive gear of the plurality of drive gears are operably coupled to one another and aligned along a first plane, and wherein a second ring gear of the plurality of ring gears and a second drive gear of the plurality of drive gears are operably coupled to one another and aligned along a second plane, vertically displaced from the first plane.

4. The instrument drive unit according to claim 1, wherein the plurality of ring gears are independently rotatable relative to one another.

5. The instrument drive unit according to any preceding claim, wherein the plurality of drive gears are vertically and horizontally offset from one another.

6. The instrument drive unit according to any preceding claim, wherein the plurality of motor gears are vertically and horizontally offset from one another.

7. The instrument drive unit according to any preceding claim, further comprising a plurality of motor shafts (40a, 40b, 40c, 40d), each motor shaft of the plurality of motor shafts extending from a corresponding motor of the plurality of motors, each motor gear of the plurality of motor gears being fixed to a corresponding motor shaft of the plurality of motor shafts.

## Patentansprüche

1. Instrumentenantriebseinheit (20) zur Verwendung in einem chirurgischen robotischen System, wobei die Instrumentenantriebseinheit umfasst:
einen Wagen (26), der dazu ausgelegt ist, mit einem robotischen Arm gekoppelt zu werden;
eine Vielzahl von Antriebswellen (66a, 66b, 66c, 66d), die drehbar in dem Schlitten gelagert sind, wobei die Vielzahl von Antriebswellen dazu ausgelegt sind, mit einem entsprechenden angetriebenen Element eines elektromechanischen chirurgischen Instruments zusammenzuwirken;
eine Vielzahl von Antriebszahnrädern (64a, 64b, 64c, 64d), wobei jedes Antriebszahnrad der Vielzahl von Antriebszahnrädern an einer entsprechenden Antriebswelle der Vielzahl von Antriebswellen befestigt ist;
eine Vielzahl von Motoren (22a, 22b, 22c, 22d); und
eine Vielzahl von Motorzahnrädern (42), wobei jedes Motorzahnrad der Vielzahl von Motorzahnrädern betriebsfähig mit einem entsprechenden Motor der Vielzahl von Motoren gekoppelt ist, wobei jedes Motorzahnrad der Vielzahl von Motorzahnrädern dazu ausgelegt ist, ein entsprechendes Antriebszahnrad der Vielzahl von Antriebszahnrädern als Reaktion auf eine Aktivierung eines entsprechenden Motors der Vielzahl von Motoren zu drehen, um eine Funktion des elektromechanischen chirurgischen Instruments zu betätigen,
ferner umfassend eine Vielzahl von Hohlrädern (62a, 62b, 62c, 62d), wobei jedes Hohlrad der Vielzahl von Hohlrädern ein entsprechendes Motorzahnrad der Vielzahl von Motorzahnrädern mit einem entsprechenden Antriebszahnrad der Vielzahl von Antriebszahnrädern betriebsfähig koppelt,
ferner umfassend eine Vielzahl von Antriebsriemen, wobei jeder Antriebsriemen der Vielzahl von Antriebsriemen (65) um ein entsprechendes Motorzahnrad der Vielzahl von Motorzahnrädern und ein entsprechendes Hohlrad der Vielzahl von Hohlrädern angeordnet ist, um die Motorzahnräder mit den Hohlrädern betriebsfähig zu koppeln,
**dadurch gekennzeichnet, dass** mindestens ein erstes Hohlrad der Vielzahl von Hohlrädern an seinem Innenumfang und an seinem Außenumfang eine Verzahnung aufweist, wobei die Verzahnung am Innenumfang des ersten Hohlrads mit einem entsprechenden Antriebszahnrad der Vielzahl von Antriebszahnrädern und die Verzahnung am Außenumfang des ersten Hohlrads mit einem entsprechenden Antriebsriemen der Vielzahl von Antriebsriemen zusammenwirkt.

2. Instrumentenantriebseinheit gemäß Anspruch 1, wobei die Vielzahl von Hohlrädern vertikal gestapelt sind.

3. Instrumentenantriebseinheit gemäß Anspruch 2, wobei ein erstes Hohlrad der Vielzahl von Hohlrädern und ein erstes Antriebszahnrad der Vielzahl von Antriebszahnrädern betriebsfähig miteinander gekoppelt und entlang einer ersten Ebene ausgerichtet sind, und wobei ein zweites Hohlrad der Vielzahl von Hohlrädern und ein zweites Antriebszahnrad der Vielzahl von Antriebszahnrädern betriebsfähig miteinander gekoppelt und entlang einer zweiten Ebene ausgerichtet sind, die vertikal von der ersten Ebene versetzt ist.

4. Instrumentenantriebseinheit gemäß Anspruch 1, wobei die Vielzahl von Hohlrädern unabhängig voneinander drehbar sind.

5. Instrumentenantriebseinheit gemäß einem der vorhergehenden Ansprüche, wobei die Vielzahl von Antriebszahnrädern vertikal und horizontal zueinander versetzt sind.

6. Instrumentenantriebseinheit gemäß einem der vorhergehenden Ansprüche, wobei die Vielzahl von Motorzahnrädern vertikal und horizontal zueinander versetzt sind.

7. Instrumentenantriebseinheit gemäß einem der vorhergehenden Ansprüche, ferner umfassend eine Vielzahl von Motorwellen (40a, 40b, 40c, 40d), wobei sich jede Motorwelle der Vielzahl von Motorwellen von einem entsprechenden Motor der Vielzahl von Motoren erstreckt, wobei jedes Motorzahnrad der Vielzahl von Motorzahnrädern an einer entsprechenden Motorwelle der Vielzahl von Motorwellen befestigt ist.

## Revendications

1. Unité d'entraînement d'instrument (20) destinée à être utilisée dans un système chirurgical robotisé, l'unité d'entraînement d'instrument comprenant :
un chariot (26) configuré pour être couplé à un bras robotisé ;
une pluralité d'arbres d'entraînement (66a, 66b, 66c, 66d) supportés de façon rotative dans le chariot, la pluralité d'arbres d'entraînement étant configurés pour réaliser une interface avec un élément entraîné correspondant d'un instrument chirurgical électromécanique ;
une pluralité de pièces dentées d'engrenage d'entraînement (64a, 64b, 64c, 64d), chaque pièce dentée d'engrenage d'entraînement de la pluralité de pièces dentées d'engrenage d'entraînement étant fixée à un arbre d'entraînement correspondant de la pluralité d'arbres d'entraînement ;
une pluralité de moteurs (22a, 22b, 22c, 22d) ; et
une pluralité de pièces dentées d'engrenage de moteur (42), chaque pièce dentée d'engrenage de moteur de la pluralité de pièces dentées d'engrenage de moteur étant fonctionnellement couplée à un moteur correspondant de la pluralité de moteurs, dans laquelle chaque pièce dentée d'engrenage de moteur de la pluralité de pièces dentées d'engrenage de moteur est configurée pour mettre en rotation une pièce dentée d'engrenage d'entraînement correspondante de la pluralité de pièces dentées d'engrenage d'entraînement en réponse à une activation d'un moteur respectif de la pluralité de moteurs pour actionner une fonction de l'instrument chirurgical électromécanique,
comprenant en outre une pluralité de pièces dentées d'engrenage couronnes (62a, 62b, 62c, 62d), chaque pièce dentée d'engrenage couronne de la pluralité de pièces dentées d'engrenage couronnes couplant fonctionnellement une pièce dentée d'engrenage de moteur correspondante de la pluralité de pièces dentées d'engrenage de moteur à une pièce dentée d'engrenage d'entraînement correspondante de la pluralité de pièces dentées d'engrenage d'entraînement,
comprenant en outre une pluralité de courroies d'entraînement, chaque courroie d'entraînement de la pluralité de courroies d'entraînement (65) étant disposée autour d'une pièce dentée d'engrenage de moteur correspondante de la pluralité de pièces dentées d'engrenage de moteur et d'une pièce dentée d'engrenage couronne correspondante de la pluralité de pièces dentées d'engrenage couronnes pour fonctionnellement coupler les pièces dentées d'engrenage de moteur aux pièces dentées d'engrenage couronnes,
**caractérisée en ce qu'**au moins une première pièce dentée d'engrenage couronne de la pluralité de pièces dentées d'engrenage couronnes a des dents d'engrenage sur une périphérie intérieure de celle-ci et sur une périphérie extérieure de celle-ci, dans laquelle les dents d'engrenage sur la périphérie intérieure de la première pièce dentée d'engrenage couronne réalisent une interface avec une pièce dentée d'engrenage d'entraînement correspondante de la pluralité de pièces dentées d'engrenage d'entraînement, et les dents d'engrenage sur la périphérie extérieure de la première pièce dentée d'engrenage couronne réalisent une interface avec une courroie d'entraînement correspondante de la pluralité de courroies d'entraînement.

2. Unité d'entraînement d'instrument selon la revendication 1, dans laquelle la pluralité de pièces dentées d'engrenage couronnes sont verticalement empilées.

3. Unité d'entraînement d'instrument selon la revendication 2, dans laquelle une première pièce dentée d'engrenage couronne de la pluralité de pièces dentées d'engrenage couronnes et une première pièce dentée d'engrenage d'entraînement de la pluralité de pièces dentées d'engrenage d'entraînement sont fonctionnellement couplées l'une à l'autre et alignées le long d'un premier plan, et dans laquelle une seconde pièce dentée d'engrenage couronne de la pluralité de pièces dentées d'engrenage couronnes et une seconde pièce dentée d'engrenage d'entraînement de la pluralité de pièces dentées d'engrenage d'entraînement sont fonctionnellement couplées l'une à l'autre et alignées le long d'un second plan, verticalement déplacé par rapport au premier plan.

4. Unité d'entraînement d'instrument selon la revendication 1, dans laquelle la pluralité de pièces dentées d'engrenage couronnes sont indépendamment rotatives les unes relativement aux autres.

5. Unité d'entraînement d'instrument selon une quelconque revendication précédente, dans laquelle la pluralité de pièces dentées d'engrenage d'entraînement sont verticalement et horizontalement décalées les unes par rapport aux autres.

6. Unité d'entraînement d'instrument selon une quelconque revendication précédente, dans laquelle la pluralité de pièces dentées d'engrenage de moteur sont verticalement et horizontalement décalées les unes par rapport aux autres.

7. Unité d'entraînement d'instrument selon une quelconque revendication précédente, comprenant en outre une pluralité d'arbres de moteur (40a, 40b, 40c, 40d), chaque arbre de moteur de la pluralité d'arbres de moteur s'étendant depuis un moteur correspondant de la pluralité de moteurs, chaque pièce dentée d'engrenage de moteur de la pluralité de pièces dentées d'engrenage de moteur étant fixée à un arbre de moteur correspondant de la pluralité d'arbres de moteur.
